# DEMANDE DE BREVET EUROPEEN

(11) **EP 0 658 625 A2**
(43) Date de publication de la demande: **21.06.1995**
(21) Numéro de dépôt: 94420331.4
(22) Date de dépôt: 25.11.1994
(51) Int. Cl.: C12N 15/29, C07K 14/415, C12N 5/04, A01H 5/00, C07K 16/16, G01N 33/53, C12Q 1/68

(54) **Protéine susceptible d'être biotinylée utilisable pour la détermination du stade de germination d'une semence**

(30) Priorité: 29.11.1993 FR 9314482; 14.02.1994 FR 9401901
(71) Demandeur: RHONE-POULENC AGROCHIMIE, 69009 Lyon (FR)
(72) Inventeur: Job, Dominique, F-69003 Lyon (FR); De Rose, Richard, F-69009 Lyon (FR); Duval, Manuel, F-69004 Lyon (FR); Job, Claudette, F-69003 Lyon (FR); Douce, Roland, F-38000 Grenoble (FR)
(74) Mandataire: Chrétien, François

(57) **Abrégé**

1. Protéine pure d'origine végétale susceptible d'être biotinylée
2. Elle est caractérisée en ce qu'elle est issue de semence d'une espèce de culture végétale, qu'elle comprend au moins une unité de 50 à 85 kDa, exprimée chez les semences et dans aucun autre organe de la plante, et disparaissant rapidement au cours des phases précoces de la germination.
3. Utilisation en agriculture comme marqueur moléculaire pour la détermination du stade de germination de semences.

## Description

La présente invention concerne un procédé d'obtention et une nouvelle protéine susceptible d'être biotinylée chez les semences matures de plantes appartenant à des espèces de légumineuses, de carotte et de betterave et son utilisation comme marqueur moléculaire de la germination de ces semences.

La germination est un processus de développement complexe, pour lequel on ne dispose à l'heure actuelle que de peu de données moléculaires précises. Ce programme de développement, au cours duquel les cellules de l'embryon passent d'un état de repos à un état d'activité métabolique intense, est mis en place essentiellement au cours de la phase d'imbibition. Il se termine, au sens physiologique, par la percée d'un organe de la plantule naissante au travers les enveloppes de la graine Bewley *et al*. (1983).

Les principales techniques utilisées pour définir la compétence des semences à germer mettent en oeuvre les tests de germination classiques, c'est-à-dire que l'on mesure, pour un lot de semences donné et dans des conditions codifiées (température, humidité, lumière, substrat), le pourcentage de germination à des temps différents après le semis. Le critère généralement utilisé pour quantifier la germination est la percée de l'enveloppe des graines par un organe de la plante naissante. La plupart des marqueurs biochimiques décrits à ce jour sont corrélés avec cette phase. Il ne s'agit donc pas de marqueurs sensu stricto de la germination, mais plutôt des phases initiales de la croissance (Fincher, 1989). Un seul exemple de marqueur précoce est, pour l'instant, bien documenté. Il concerne la germine des céréales, protéine de l'embryon, dont la cinétique d'apparition suit de très prés la cinétique d'imbibition (Lane et al., 1992). Il faut noter que la phase initiale d'imbibition est réversible jusqu'à un certain point. Suite à une hydratation contrôlée des graines, on peut les sécher, tout en conservant leur intégrité biologique et leur capacité germinative. Dès que la plantule apparaît, l'engagement de celle-ci dans sa croissance devient irréversible. En effet, une déshydratation à ce stade entraîne irrémédiablement la mort des plantules (Bewley & Black, 1983).

Les procédés de pré-germination ("priming") développés par les sociétés de semences sont basés sur la réversibilité de la phase initiale d'imbibition. Les semences sont en général hydratées de manière contrôlée, puis elles sont séchées (Karsen et al., 1989 ; Tarquis & Bradford, 1992). Ces procédés apportent une réelle valeur ajoutée aux semences car ils permettent :
1) d'homogénéiser les lots de semences par rapport à la germination,
2) un gain de temps appréciable pour la levée après le semis, car un certain nombre de processus biochimiques nécessaires à l'accomplissement de la germination seraient déjà réalisés au cours du priming,
3) une amélioration de la qualité germinative de lots de semences âgées, probablement en raison du fait que des mécanismes de réparation de structures biologiques endommagées au cours de la maturation finale des semences se mettent en place au cours du priming.

Ne disposant pas de marqueurs d'étapes précoces de germination, l'optimisation de tels procédés repose uniquement sur la conduite de tests de germination, qui demandent plusieurs jours d'expérimentation. De plus, si le traitement échoue (les lots de semences étant par nature hétérogènes, il faut donc optimiser le traitement pour chacun des lots), le lot est perdu. Il existe donc un besoin important de trouver un marqueur moléculaire facile à détecter et la possibilité de suivre en continu la phase d'imbibition, par l'intermédiaire d'un tel marqueur moléculaire, constituerait donc un progrès considérable, permettant d'adapter le priming à chaque lot de semences.

Les cellules végétales sont capables de synthétiser les principales vitamines. L'une d'entre elles, la biotine, sert de cofacteur à un petit nombre d'enzymes jouant un rôle primordial dans le métabolisme cellulaire, connues sous le nom de carboxylases à biotine (Knowles, 1989 ; Wurtele & Nikolau, 1990 ; Alban et al., 1993) : acétyl-CoA carboxylase (EC 6.4.1.2), 3-méthylcrotonyl-CoA carboxylase (EC 6.4.1.4), propionyl-CoA carboxylase (EC 6.4.1.3) et pyruvate carboxylase (EC 6.4.1.1). L'étude de ces protéines est donc d'importance majeure pour comprendre le redémarrage du métabolisme lors de la germination des semences. L'acétyl-CoA carboxylase est, chez les plantes, la plus étudiée des enzymes à biotine, car elle constitue la cible d'herbicides puissants chez les monocotylédones (Hoppe & Zacher, 1985 ; Burton et al., 1987a,b). Cette enzyme joue en effet un rôle clé dans la synthèse des acides gras. Le rôle des trois autres carboxylases à biotine reste pour l'instant inconnu chez les plantes. On sait que les semences à réserves lipidiques (Stumpf, 1980 ; Harwood, 1988), ainsi que les semences de pois (Bettey et ai., 1992), contiennent une activité acétyl-CoA carboxylase, vraisemblablement impliquée dans la synthèse des triglycérides de réserve. Par contre, on ne sait pas si les semences renferment d'autres protéines biotinylées, et si elles jouent un rôle au cours de la germination.

La présente invention a donc pour objet une protéine pure d'origine végétale susceptible d'être biotinylée, caractérisée en ce qu'elle est issue de semence d'une espèce de culture végétale, qu'elle comprend au moins une unité de 50 à 85 kDa, est exprimée chez les semences et dans aucun autre organe de la plante, et disparaît rapidement au cours des phases précoces de la germination.

La présente invention a plus particulièrement pour objet une protéine pure, susceptible d'être biotinylée et issue de semence de légumineuses, par exemple le pois, le haricot, le lupin, la luzerne, le soja, la lentille, mais aussi chez d'autres espèces comme par exemple les ombellifères telles que par exemple la carotte ou encore les chenopodiacées telles que par exemple la betterave. Dans le cas du pois, la protéine est dénommée SBP65. Elle concerne aussi les protéines équivalentes à cette dernière, qui établissent une interaction avecla biotine.

L'invention concerne également de nouveaux anticorps, caractérisés en ce qu'ils reconnaissent la protéine SBP65.

Elle concerne encore des sondes moléculaires, caractérisées en ce qu'elles sont dérivées de la protéine SBP65 ou des protéines équivalentes;
Elle concerne également l'utilisation de la spécificité d'expression tissulaire et du patron de développement de ces marqueurs afin de mesurer le plus précisément possible l'état d'avancement de la germination, plus particulièrement dans la phase précoce d'imbibition, en particulier leur utilisation comme marqueur moléculaire, protéique et nucléique, de la germination chez les semences de légumineuses, par exemple le pois, le haricot, le lupin, la luzerne, le soja, la lentille, mais aussi chez d'autres espèces comme par exemple les ombellifères telles que par exemple la carotte ou encore les chenopodiacées telles que par exemple la betterave. La détection de ces marqueurs peut se faire soit par détection avec des anticorps spécifiques, dans le cas d'utilisation dans les légumineuses, soit directement à l'aide de révélation colorée de la biotine dans le cas d'autres cultures telles que la carotte ou la betterave.

Cette détection peut être faite à l'aide d'un dispositif(kit), qui fait aussi partie de l'invention.

L'invention a également pour objet un procédé pour la transformation de cellules de plantes par des séquences d'ADN codant pour la protéine SBP65 ou une protéine équivalente.

De même elle concerne un procédé pour la transformation de cellules de plantes par des séquences dL'ADNcodant pour un ARN anti-sens de la protéine SBP65, ou toute protéine biotinylée équivalente du point de vue du patron de développement et de l'expression tissulaire , pour inhiber la synthèse de telles protéines, et ainsi créer des plantes stériles.

L'invention a encore pour objet un procédé de transformation des cellules de plantes par des séquences d'ADN exprimant un ARN codant pour une protéine qui pourrait différer de SBP65 par sa séquence et son mode d'interaction avec la biotine, mais dont la construction permettrait d'assurer une spécificité tissulaire d'expression analogue à celle de la protéine SBP65 et la possibilité de piéger, dans les semences en développement, la biotine libre néo-synthétisée et/ou absorbée du sol par la plante, dans le but de créer des plantes stériles.

L'invention a enfin pour objet les cellules végétales transformées selon les procédés ci-dessus ainsi que les plantes transformées obtenues par régénération de ces cellules transformées.
Caractérisation de protéines susceptibles d'être biotinylées:
On utilise la semence de pois (Pisum sativum cv. Douce Provence) comme modèle. Grâce à l'utilisation de techniques ELISA et d'un marquage spécifique avec la streptavidine [protéine bactérienne douée d'une très haute affinité spécifique pour la biotine (Green, 1990)] couplée à la peroxydase (Sigma), les protéines biotinylées sont aisément décelables dans un extrait total de protéines réalisé à partir d'une seule semence. Cette quantification n'exige qu'un petit nombre d'étapes faciles à mettre en oeuvre : broyage des graines dans un mixer type Waring blender (30 sec), reprise de la poudre dans un tampon d'homogénéisation (Hepes pH 8,0, contenant divers inhibiteurs de protéases ; Alban et al., Plant Physiol. 102, 957-965, 1993), centrifugation (15 min à 20 000 g en tubes de centrifugation type Eppendorf) pour éliminer les débris cellulaires, réalisation d'essais reposant sur les techniques classiques ELISA.

Les protéines, susceptibles d'être biotinylées, de l'extrait brut peuvent être également facilement repérées, non plus en mélange, mais individuellement, suite à la séparation des protéines de l'extrait total en gel de polyacrylamide en présence de dodécylsulfonate de sodium (SDS), électro-transfert des protéines sur membrane de nitrocellulose et révélation spécifique avec la streptavidine couplée à la peroxydase (Nikolau et al., 1985; Alban et al., Plant Physiol. 102, 957-965,1993). Cette dernière technique a été optimisée dans le but de permettre l'utilisation de matériels de microanalyse électrophorétique de protéines en gels de polyacrylamide préformés (PhastSystem et PhastTransfer de Pharmacia), qui présentent l'avantage de conduire l'analyse des échantillons de manière très rapide.

Utilisant ces méthodes, nous avons observé que les semences matures de Pois renferment une protéine majoritaire susceptible d'être biotinylée, de poids moléculaire 65 kDa.

Des mesures d'activité enzymatique effectués comme décrit par Alban et al. (1993) montrent que l'extrait brut renferme deux activités carboxylase à biotine: l'acétyl-CoA carboxylase et la 3-méthylcrotonyl-CoA carboxylase. Les activités propionyl-CoA carboxylase et pyruvate carboxylase ne sont pas détectables.

La protéine SBP65 a été purifiée à l'état d'homogénéité à partir d'un extrait de semences de pois réalisé comme décrit ci-dessus, et en utilisant une technique de chromatographie sur colonne d'affinité constituée d'avidine-Sepharose (Kohanski & Lane, 1990 ; Alban *et al*., 1993) [l'avidine est une protéine d'oeuf de poule qui, comme la streptavidine bactérienne, est douée d'une très haute affinité spécifique pour la biotine (Green, 1990]. Cette méthode permet la purification de l'ensemble des protéines susceptible d'être biotinylées contenues dans l'extrait brut. Dans une étape ultérieure, on peut, en effectuant une chromatographie d'échange d'ions sur une colonne Mono-Q HR 5/5 (Pharmacia), séparer ces protéines en deux fractions distinctes. L'une, non retenue par la colonne, contient la protéine SBP65 pure. L'autre, retenue par cette colonne, est éluée en présence de KCl 0.3 M et contient les deux principales activités carboxylases à biotine présentes dans l'extrait brut: l'acétyl-CoA carboxylase et la 3-méthylcrotonyl-CoA carboxylase.

Les résultats principaux de cette purification sont les suivants:
1) La protéine SBP65 ne porte aucune des activités carboxylase à biotine(EC 6.4.1.1, EC 6.4.1.2, EC 6.4.1.3, et EC 6.4.1.4) décrites chez les micro-organismes, la levure et les eucaryotes (Knowles, 1989). Elle contient une mole de biotine par mole de polypeptide de 65 kDa, la fixation de la biotine sur la protéine étant de nature forte, notamment ionique forte ou covalente. Son poids moléculaire, estimé par filtration sur gel (Sephacryl S-300 HR, Pharmacia), est sous la forme native, de 450 ± 60 kDa. Ceci indique que la forme native de la protéine SBP65 correspond à l'association de six à huit sous-unités identiques, chacune ayant un poids moléculaire de 65 kDa.
2) L'activité acétyl-CoA carboxylase est portée par un polypeptide biotinylé de 200 kDa, ainsi que décrit par Bettey et al. (1992).
3) L'activité 3-méthylcrotonyl-CoA carboxylase est portée par deux polypeptides, constituant les deux sous-unités de l'enzyme : l'un biotinylé de 75 kDa et l'autre non biotinylé de 50 kDa, en accord avec les résultats de Alban et al. (1993) concernant la purification à l'état d'homogénéité de cette enzyme à partir de la feuille de Pois.

SBP65 correspond donc à une nouvelle protéine susceptible d'être biotinylée d'origine végétale.

### Distribution tissulaire de la protéine

La purification à l'état d'homogénéité de la protéine SBP65 a permis, par immunisation d'un lapin, l'obtention d'anticorps spécifiques nouveaux, qui font également partie de l'invention. L'utilisation de ces anticorps montre que l'expression de la protéine est spécifique des semences. Elle n'est détectée dans aucun autre organe de la plante (feuilles, tiges, racines, gousses et fleurs), quelque soit l'état de développement de la plante. Une telle spécificité tissulaire n'est pas retrouvée pour les carboxylases à biotine. Les activités acétyl-CoA carboxylase et 3-méthylcrotonyl-CoA carboxylase sont en effet détectables dans tous les organes de la plante.

### Clonage de l'ADNc codant pour la protéine

Les anticorps anti-SBP65 ont été utilisés pour cribler une banque d'ADNc, correspondant aux ARNm polyadénylés isolés de semences de pois. Un clone bactérien recombinant( hôte: *Escherichia coli* K 12; système de clonage: predigested lambda ZAP® II/ *Eco* RI cloning kit, Stratagene) , exprimant une protéine reconnue par les anticorps anti-SBP65, a été isolé et l'ADNc ainsi cloné a été caractérisé. Sa longueur est de l'ordre de 2000 bases.

Des expériences de séquençage montrent que l'ADNc comporte dans le sens de la traduction (Séquences A et C) :
1) une séquence consensus d'initiation de la traduction chez les plantes : ATCAATGGC,(souligné dans les séquences A et C)
2) un signal consensus de polyadénylation : AATAAA,(souligné dans les séquences B et C)
3) une queue poly(A) constituée de 18 résidus A.

De plus, l'extrémité 5' de cet ADNc contient des motifs de séquence correspondant exactement à ceux que nous avons déterminés par séquençage de la protéine à partir de peptides obtenus par coupure de SBP65 avec le bromure de cyanogène et la trypsine. Ces motifs de séquence correspondent à 44 résidus d'acides aminés, localisés à l'extrémité N terminale de SBP65(indiqués en gras dans les séquences A et C). Des expériences de séquençage de la protéine SBP65 ont en outre permis d'identifier la Lysine 103 comme représentant le résidu d'amino-acide portant la biotine.

La séquence C est la séquence complète de l'ADNc codant pour la protéine selon l'invention et qui fait également partie de l'invention: sa longueur est de 1969 nucléotides. La région codante pour la protéine entière est de 1653 nucléotides, du nucléotide 51 au nucléotide 1703. La séquence soulignée des nucléotides 47 au 55 correspond à la séquence consensus retrouvée au niveau du codon initiateur des plantes dicotylédones (AACAATGGC). L'autre séquence soulignée des nucléotides 1828 au 1838 correspond à la séquence signal de polyadénylation. La séquence c présente également la séquence protéique traduite à partir de cet ADN c, comportant 551 résidus. En gras, les séquences peptidiques obtenues par microséquençage. Le résidu Lysine, en position 103 (souligné) est celui liant la biotine de manière covalente(résidu biocytine).

La comparaison des séquences nucléotidiques et protéiques obtenues pour la protéine SBP65 avec celles contenues dans les banques Swiss-Prot et Gene Bank ne met en évidence aucune homologie avec une protéine connue à ce jour.

### Présence de protéines équivalentes à SBP65 chez d'autres espèces de semences

L'utilisation des anticorps anti-SBP65 montre que la protéine est présente dans différentes variétés de semences de pois (Cador, Finale, Cash, Progreta, Twigy), et chez différentes légumineuses (Haricot, Soja, Lentille, Lupin, Luzerne). Pour les espèces n'appartenant pas à la famille des légumineuses, la réactivité des anticorps anti-SBP65 est faible. Toutefois, dans le cas de la Carotte, deux protéines biotinylées majoritaires de 62 ± 2 kDa et de 30 ± 2 kDa sont détectées dans les semences matures, correspondant probablement aux polypeptides préalablement mis en évidence chez l'embryon somatique (Wurtele & Nikolau, 1992; Caffrey et al., 1993). Ces protéines disparaissent très rapidement au cours de la germination, avant que l'apparition de la radicule ne soit observée. Le même type de résultats est obtenu dans le cas de la betterave. Là encore une protéine biotinylée de 62 ± 2 kDa est facilement reconnaissable dans des extraits bruts de semences sèches, disparaissant à une vitesse élevée au cours des phases précoces de la germination.

### EXEMPLE 1.Evolution des protéines totales et de la protéine SBP65 au cours de la germination de semences de pois.

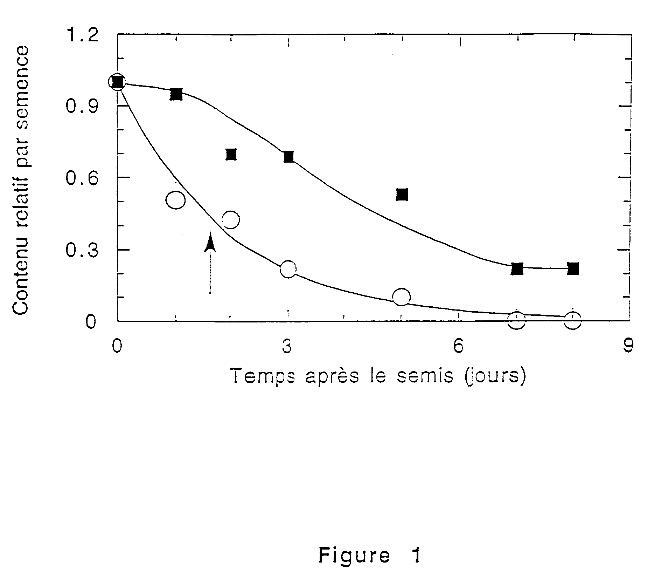

Les expériences de germination sont réalisées en serre, à 20°C, et en lumière contrôlée (photopériode 12 h, lumière blanche en tubes fluorescents, 10-40 µE m⁻² s⁻¹). Les semences de pois matures (var Douce Provence) sont mises à germer sur terreau au temps zéro ; elles sont arrosées chaque jour avec de l'eau. Les semences sont prélevées en fonction du temps. La flèche indique la percée radiculaire. Pour chaque échantillon, un extrait brut est réalisé comme indiqué. La figure 1 représente une courbe montrant l'évolution, dans le temps, du contenu relatif ( 1 = 100%) des semences en protéines totales (■) [i.e. les protéines de réserve, protéines majoritaires des semences (Bewley & Black, 1983)], et en protéine SBP65 (○), cette dernière étant révélée spécifiquement en conduisant des tests ELISA avec les anticorps anti-SBP65. Les résultats sont présentés sous forme normalisée par rapport aux mesures effectuées avec les semences matures (temps zéro).On peut constater que la protéine SBP65 disparaît très rapidement au cours de la germination. Fait remarquable, une partie considérable du contenu initial (de l'ordre de 60%) disparaît avant que ne soit observée la percée radiculaire (celle ci est indiquée par une flèche verticale). La cinétique de disparition de SBP65 est ainsi beaucoup plus rapide que celle des protéines de réserve de la semence. On sait que la mobilisation de ces réserves commence, lorsque la radicule perce les enveloppes (Bewley *et al*, 1983). Ces résultats démontrent que SBP65 est un marqueur des phases précoces de la germination.

### EXEMPLE 2:

En complément de cette étude sur la germination, on étudie, (en utilisant la méthode décrite par Baldet et al., 1993) l'évolution dans le temps de l'expression de la protéine SBP65 et de la biotine libre, c'est-à-dire de la vitamine non complexée à des protéines, au cours de la maturation des semences de pois. On sait que les cellules végétales ont l'équipement enzymatique nécessaire à la biosynthèse de cette vitamine, et que, de plus, la vitamine sous sa forme libre se retrouve, dans les tissus végétatifs comme les feuilles, en excès par rapport à la biotine liée à des protéines (Baldet et al., 1993). Les principaux résultats obtenus sont les suivants :
1) Dans les très jeunes semences, la protéine SBP65 n'est pas encore présente, la biotine libre est toujours en excès par rapport à la biotine liée.
2) Le niveau maximum de la protéine SBP65 est détecté en phase finale de maturation des semences, en même temps que s'accumulent les principales substances de réserve, protéines, amidon, triglycérides, et que les semences entrent en phase de déshydratation. A ce stade de développement, la biotine liée (c'est-à-dire la biotine principalement liée à la protéine SBP65, puisque celle-ci devient, à ce stade, la protéine biotinylée majeure des semences) est en excès par rapport à la biotine libre.

L'ensemble de ces résultats montrent le rôle biologique de la protéine SBP65 :
1) Elle constitue une réserve de biotine, utilisée dans la germination pour le redémarrage du métabolisme.
2) La protéine SBP65 régule le niveau de biotine libre de la cellule embryonnaire.

SEQUENCES

A

GAATTCGAGG ATCCGGGTAC CATGGTTTTT TTTTTTTTCA TAACCAATAC 50

AGAGAAAAAC GCACATTCAT TATCAATGGC ATCTGAACAA TTATCTCGCA 100
MetAl aSerGluGln LeuSerArgA
GAGAAAACAT CACAACTGAG AGAAAGATTC AAAACGCGGA AGACAGTGTC 150
rgGluAsnIl eThrThrGlu ArgLysIleG lnAsnAlaGl uAspSerVal
CCTCAAAGGA CAACCCACTT CGAGCTTAGA GAGACCCACG AACTTGGACC 200
ProGlnArgT hrThrHisPh eGluLeuArg GluThrHisG luLeuGlyPr
AAACTTTCAG TCTCTCCCTC GCAACGAGAA TCAAGCTTAC CTTGACCGTG 250
oAsnPheGln SerLeuProA rgAsnGluAs nGlnAlaTyr LeuAspArgG
GTGCACGTGC TCCTTTGAGT GCAAATGTAT CAGAAAGTTA CCTTGATCGT 300
lyAlaArgAl aProLeuSer AlaAsnValS erGluSerTy rLeuAspArg
GCACGTGTTC CTTTGAATGC AAATATACCA GAACACAGAG TTAGAGAAAA 350
AlaArgValP roLeuAsnAl aAsnIlePro GluHisArgV alArgGluLy
AGAAGATTTT GGTGGTGTTC GTGATATGGG AAAGTTTCAG ATGGAATCGA 400
sGluAspPhe GlyGlyValA rgAspMetGl yLysPheGln MetGluSerL
AAGGAGGGAA TAAGAGTTTG GCCGAAGATA GAGAAACTCT CGATACACGA 450
ysGlyGlyAs nLysSerLeu AlaGluAspA rgGluThrLe uAspThrArg
TCTAGAATGG TTACTGGAAC ACCTCACATT AAAGAAGCAT CGGGAAAAGG 500
SerArgMetV alThrGlyTh rProHisIle LysGluAlaS erGlyLysGl
ACAAGTTGTG GAGGAAAGAG AGAGAGCGAG 530
yGlnValVal GluGluArgG luArgAla

B

GTCGACAGTG GATGGAACTA GGGCTGCTGC GAATGCTGTT GAAGGAGCGG 50
TTGGGTATGT TGCACCTAAA GCTTCTGAGC TTGCGGCGAA ATCGGTGGAA 100
ACTGTTAAGG GTTTGGCTGC TTCTGCTGGT GAAACTGCTA AGGAGTTTAC 150
TGCTAGGAAG AAAGAAGAAT CATGGCGGGA ATATGAGGCT AAAAGGGCTT 200
CTCAACTTCA GGAAGGTGAA GAAATCTTGC CATCTACCGG AGGTATCGGA 250
AAAGTGTTAC CCAGTGGAGA AAGAACTCAA GCACAAGGAA CCAATCTTCA 300
AGAGAAGGTA CAAGGAAAAG GAAGTGATAT ATTAGGAGCT GTGACTGAAA 350
CTGTGAGTGA CATTGGAAGT AGCATGATTA AACCAATAGA TAATGCTAAT 400
ACTAAAGTTA AGGAACATGG TGGCACTACT ATTACACCAA AAGGACAAGA 450
TGCTGGTGGT GTTTTGGATG CTATTGGTGA AACTATAGCT GAGATTGCAC 500
ATACAACTAA AGTCATTGTT GTTGGTGAAG ATGATGAAGT AGAAAAGTCA 550
ATGCAGAAGA ATATTGGGTC AGATTCTCAC TCTCTTGATC GTGCCAAGCA 600
TGAAGGATAT AGAGCACCAA AGAATAATGT TTCTTAATTC CAAAGTTTGA 650
AGACAATGAA TGTGTTTGTT TGATGCAGAA GTTTAGTAAT ATGTTAATCT 700
TAATTAGCTG TCAGTGAAGA AGTTCAATGT TTTGTGGCTT TGTTTTATGG 750
AGTTGTGTGA ATAAATTACA ATCTCATTCT TGAGATTGTC AATAATAGCA 800
AATATATCTT ATGCTTATGT CTTTTGTAAG TCAATGTTGT AATGTAATAA 850
TATATACTTT TATTTAATAT TCTGTTATTG CTAAAAAAAA AAAAAAAAAA 900
CCATGGTACC CGGATCCTCG AATTC 925

TTTTTTTTTT TTTCATAACC AATACAGAGA AAAACGCACA TCCATTATCA ATGGCATCTG AACAATTATC TCGCAGAGAA AACATCACAA CCGAGAGAAA GATTCAAAAC GCGGAAGACA 120
MetAlaSerG luGlnLeuSe rArgArgGlu AsnIleThrT hrGluArgLy sIleGlnAsn AlaGluAspS
GTGTCCCTCA AAGGACAACC CACTTCGAGC TTAGAGAGAC CCACGAACTT GGACCAAACT TTCAGTCTCT CCCTCGCAAC GAGAATCAAG CTTACCTTGA CCGTGGTGCA CGTGCTCCTT 240
erValProGl nArgThrThr HisPheGluL euArgGluTh rHisGluLeu GlyProAsnP heGlnSerLe uProArgAsn GluAsnGlnA laTyrLeuAs pArgGlyAla ArgAlaProL
TGAGTGCAAA TGTATCAGAA AGTTACCTTG ATCGTGCACG TGTTCCTTTG AATGCAAATA TACCAGAACA CAGAGTTAGA GAAAAAGAAG ATTTTGGTGG TGTTCGTGAT ATGGGAAAGT 360
euSerAlaAs nValSerGlu SerTyrLeuA spArgAlaAr gValProLeu AsnAlaAsnI leProGluHi sArgValArg GluLysGluA spPheGlyGl yValArgAsp MetGlyLysP
TTCAGATGGA ATCGAAAGGA GCGAATAAGA CTTTGGCCGA AGATAGAGAA ACTCTCGATA CACGATCTAG AATGGTTACT GGAACACCTC ACATTAAAGA AGCATCGGGA AAAGGACAAG 480
heGlnMetGl uSerLysGly GlyAsnLysS erLeuAlaGl uAspArgGlu ThrLeuAspT hrArgSerAr gMetValThr GlyThrProH isIleLysGl uAlaSerGly LysGlyGlnV
TTGTGGAGGA AAGAGAGAGA GCGAGAGAAA GAGCAATGGA AGAAGAAGAG AAAAGGTTAA CAATGGAAGA GATATCGAAG TATAGAAACC AAGCTCAACA AAGTGCATTG GAAGCGCTTT 600
alValGluGl uArgGluArg AlaArgGluA rgAlaMetGl uGluGluGlu LysArgLeuT hrMetGluGl uIleSerLys TyrArgAsnG lnAlaGlnGl nSerAlaLeu GluAlaLeuS
CAGCAGCACA AGAGAAATAC GAAAGAGCGA AACAAGCAAC AAATGAAACA CTACGCACAA CGACACAGGC TGCACAAGAG AAAGGAGAAG CAGCACAAGC GAAAGATGCA ACTTTTGAGA 720
erAlaAlaGl nGluLysTyr GluArgAlaL ysGlnAlaTh rAsnGluThr LeuArgThrT hrThrGlnAl aAlaGlnGlu LysGlyGluA laAlaGlnAl aLysAspAla ThrPheGluL
AAACACAACA AGGTTATGAA ATGACAGGAG ACACAGTTTC AAATTCTGCA AGAACTGCTT CTGAGAAAGC AGCACAGGCT AAAAATACAA CTCTTGGAAA GACACAACAA GGTTATGAGG 840
ysThrGlnGl nGlyTyrGlu MetThrGlyA spThrValSe rAsnSerAla ArgThrAlaS erGluLysAl aAlaGlnAla LysAsnThrT hrLeuGlyLy sThrGlnGln GlyTyrGluA
CAACAAGAGA CACAGTTTCA AATGCTGCAA GAACTGCGGC GGAGTATGCT ACTCCTGCTG CGGAGAAAGC CAGGTGTGTG GCTGTTCAGG CGAAAGATGT TACTCTGGAA ACAGGTAAGA 960
laThrArgAs pThrValSer AsnAlaAlaA rgThrAlaAl aGluTyrAla ThrProAlaA laGluLysAl aArgCysVal AlaValGlnA laLysAspVa lThrLeuGlu ThrGlyLysT
CAGCGGCGGA GAAAGCCAAG TGTGCCGCGG AAATTGCTGC GTTGATTTGA AGGAGAAGGC CACTGTGGCA GGGTGGACTG CGTCGCATTA TGCCACACAG TTGACAGTGG 1080
hrAlaAlaGl uLysAlaLys CysAlaAlaG luIleAlaAl aValAspLeu LysGluLysA laThrValAl aGlyTrpThr AlaSerHisT yrAlaThrGln LeuThrValA
ATGGAACTAG CGCTGCTGCG AATGCTGTTG AAGGAGCGGT TGGGTATGTT GCACCTAAAG CTTCTGAGCT TCGGCGGAAA CTGTTAAGGG TTTGGCTCTG TCTGCTGGTG 1200
spGlyThrAr gAlaAlaAla AsnAlaValG luGlyAlaVa lGlyTyrVal AlaProLysA laSerGluLe uAlaAlaLys SerValGluT hrValLysGl yLeuAlaAla SerAlaGlyG
AAACTGCTAA GGAGTTTACT GCTAGGAAGA AAGAAGAATC ATGGCGGGAA TATGAGGCTA AAAGGGCTTC TCAACTTCAG GAAGGTGAAG AAATCTTGCC ATCTACCGGA GGTATCGGAA 1320
lnThrAlaLy sGluPheThr AlaArgLysL ysGluGluSe rTrpArgGlu TyrGluAlaL ysArgAlaSe rGlnLeuGln GluGlyGluG luIleLeuPr oSerThrGly GlyIleGlyL
AAGTGTTACC CAGTGGAGAA AGAACTCAAG CACAACGAAC CAATCTTCAA GAGAACGTAC AAGGAAAAGG AAGTCATATA TTAGGAGCTG TGACTGAAAC TGTGAGTGAC ATTGGAAGTA 1440
ysValLeuPr oSerGlyGlu ArgThrGlnA laGlnArgTh rAsnLeuGln GluAsnValG lnGlyLysGl ySerAspIle LeuGlyAlaV alThrGluTh rValSerAsp IleGlySerS
GCATGATTAA ACCAATAGAT AATGCTAATA CTAAAGTTAA GGAACATGGT GGCACTACTA TTACACCAAA AGGACAAGAT GCTGGTGGTG TTTTGGATGC TATTGGTGAA ACTATAGCTG 1560
erMetIleLy sProIleAsp AsnAlaAsnT hrLysValLy sGluHisGly GlyThrThrI leThrProLy sGlyGlnAsp AlaGlyGlyV alLeuAspAl aIleGlyGlu ThrIleAlaG
AGATTGCACA TACAACTAAA GTCATTGTTG TTGGTGAAGA TGATGAAGTA GAAAAGTCAA TGCAGAAGAA TATTGGGTCA GATTCTCACT CTCTTGATCG TGCCAAGCAT GAAGGATATA 1680
luIleAlaHi sThrThrLys ValIleValV alGlyGluAs pAspGluVal GluLysSerM etGlnLysAs nIleGlySer AspSerHisS erLeuAspAr gAlaLysHis GluGlyTyrA
GAGCACCAAA GAATAATGTT TCTTAATTCC AAAGTTTGAA GACAATGAAT GTGTTGTTTT GATGCAGAAG TTTAGTAATA TGTTAATCTT AATTAGCTGT CAGTGAGAAG GTTCAATGTT 1800
rgAlaProLy sAsnAsnVal Ser
TTGTGGCTTT GTTTTATGGA GTTGTGTGAA TAAATTACAA TCTCATTCTT GAGATTGTCA ATAATAGCAA ATATATCTTA TGCTTATGTC TTTTGTAAGT CAATGTTGTA ATGTAATAAT 1920
ATATACTTTT ATTTAATATT CTGTTATTGC TAAAAAAAAA AAAAAAAAA 1969

### REFERENCES BIBLIOGRAPHIQUES

Alban, C., Baldet, P., Axiotis, S. & Douce, R. (1993) Plant Physiol. 102, 957-965
Baldet, P., Alban, C., Axiotis, S. & Douce, R. (1993) Arc. Biochem. Biophys. 303, 67- 73
Bewley *et al*, (1983) *in* Physiology and Biochemistry of Seeds in Relation to Germination, Vol. 1, pp. 177-244, Springer-Verlag, Berlin
Burton, J. D., Gronwald, J. W., Somers, D. A., Connelly, J. A., Gengenbach, B. G. & Wyse, D. L. (1987a) Biochem. Biophys. Res. Commun. 148, 1039-1044
Burton, J. D., Gronwald, J. W., Somers, D. A., Gengenbach, B. G. & Wyse, D. L. (1987b) Pestic. Biochem. Physiol. 34, 76-85
Caffrey, J. J., Keller, G., Wurtele, E. S. & Nikolau, B. J. (1993) Plant Physiol. 102, Abstract 524
Fincher, G. B. (1989) Annu. Rev. Plant Physiol. Plant Mol. Biol. 40, 305-346
Green, N. M. (1990) Methods Enzymol. 184, 51-67
Hoppe, H. H. & Zacher, H. (1985) Pestic. Biochem. Physiol. 24, 298-305
Karsen, C. M., Haigh, A., van der Toorn, P. & Weges, R. (1989) *in* Recent Advances in the Development and Germination of Seeds (Taylorson, R. B., ed.) NATO ASI series, Series A, Life sciences, vol. 187, pp. 269-280
Knowles, J. R. (1989) Annu. Rev. Biochem. 58, 195-221
Kohanski, R. A. & Lane, M. D. (1990) Methods Enzymol. 184, 194-200
Lane, B. G., Cuming, A. C., Frégeau, J., Carpita, N. C., Hurkman, W. J., Bernier, F., Dratewa-Kos, E. & Kennedy, T. D. (1992) Eur. J. Biochem. 209, 961-969
Nikolau, B. J., Wurtele, E. S. & Stumpf, P. K. (1985) Anal. Biochem. 149, 448-453
Motel, A., Günther, S., Clauss, M., Kobek, K., Focke, M. & Lichtenthaler, H. K. (1993) Z. Naturforsch. 48c, 294-3000
Shellhammer, J. & Meinke, D. (1990) Plant Physiol. 93, 1162-1167
Schneider, T., Dinkins, R., Robinson, K., Shellhammer, J. & Meinke, D. W. (1989) Dev. Biol. 131, 161-167
Tarquis, A. M. & Bradford, K. J. (1992) J. Exp. Bot. 43, 307-317
Wurtele, E.S. & Nikolau, B. J. (1990) Arch. Biochem. Biophys. 278, 179-186
Wurtele, E. S. & Nikolau, B. J. (1992) Plant Physiol. 99, 1699-1703

## Revendications

1. Protéine pure d'origine végétale susceptible d'être biotinylée, caractérisée en ce qu'elle est issue de semence d'une espèce de culture végétale, qu'elle comprend au moins une unité de 50 à 85 kDa, est exprimée chez les semences et dans aucun autre organe de la plante, et disparaît rapidement au cours des phases précoces de la germination.

2. Protéine selon la revendication 1, caractérisée en ce qu'elle est issue d'une espèce d'ombellifère.

3. Protéine selon la revendication 2, caractérisée en ce qu'elle est issue de la carotte.

4. Protéine selon la revendication 1, caractérisée en ce qu'elle est issue d'une espèce de chenopodiacée.

5. Protéine selon la revendication 4, caractérisée en ce qu'elle est issue de la betterave.

6. Protéine selon la revendication 1, caractérisée en ce qu'elle est de plus reconnue par l'anticorps de la semence de pois, dénommée SPB65 et issue d'une légumineuse.

7. Protéine selon 6, caractérisée en ce qu'elle est issue de semence de pois et qu'elle comprend au moins une unité de 65 kDa.

8. Protéine selon 1, caractérisée en ce que:
a) La protéine SBP65 ne porte aucune des activités carboxylase (EC 6.4.1.1, EC 6.4.1.2, EC 6.4.1.3, et EC 6.4.1.4). Elle contient une mole de biotine par mole de polypeptide de 65 kDa, la fixation de la biotine sur la protéine étant de nature forte, covalente. Son poids moléculaire, est sous la forme native, de 450 ± 60 kDa. Cequi correspond à l'association de six à huit sous-unités identiques, chacune ayant un poids moléculaire de 65 kDa.
b) L'activité acétyl-CoA carboxylase est portée par un polypeptide biotinylé de 200 kDa,
c) L'activité 3-méthylcrotonyl-CoA carboxylase est portée par deux polypeptides, constituant les deux sous-unités de l'enzyme : l'un biotinylé de 75 kDa et l'autre non biotinylé de 50 kDa, confirmant la purification à l'état d'homogénéité de cette enzyme à partir de la feuille de pois.

9. Protéine selon la revendication 8, caractérisée en ce qu'elle comprend dans la structure de son ARN m les terminaisons 5' et 3' selon les séquences A, B et C

10. Protéine biotinylée, caractérisée en ce qu'elle comprend une protéine selon 1. à 9.

11. ADNc, d'environ 2kb, codant pour la protéine selon l'une des revendications 1.à 9, caractérisé en ce qu'il a la séquence suivante, comportant, dans le sens de la traduction, la terminaison 5' selon la Séquence A et la terminaison 3' selon la séquence B):
a) une séquence consensus d'initiation de la traduction chez les plantes : ATCAATGGC,
b) un signal consensus de polyadénylation : AATAAA,
c) une queue poly(A) constituée de 18 résidus A.
l'extrémité 5' de cet ADNc contenant en outre des motifs de séquence correspondant à 44 résidus d'acides aminés, localisés à l'extrémité N terminale de SBP65 et correspondant exactement à ceux déterminés par séquençage de la protéine à partir de peptides obtenus par coupure de SBP65 avec le bromure de cyanogène et la trypsine.

12. ADNc, d'environ 2kb, codant pour la protéine selon l'une des revendications 1.à 9, caractérisé en ce qu'il a la séquence C

13. Anticorps, caractérisés en ce qu'ils reconnaissent la protéine selon l'une des revendications 1, 6, 7 8, 9 et 10.

14. Sondes moléculaires, caractérisées en ce qu'elles sont dérivées de la protéine selon l'une des revendications 1 à 9, ou de l'ADNc selon les revendications 11 et 12.

15. Utilisation de protéines susceptibles d'être biotinylées selon l'une des revendications 1 à 9 ou de sondes selon la revendication 14, comme marqueur moléculaire pour la détermination du stade de germination de semences.

16. Dispositif (kit) pour la détermination du stade de germination de semences, caractérisé en ce qu'il est basé sur la détection de la biotine ou la reconnaissance par des anticorps selon la revendication 13, utilisables dans une technique ELISA.

17. Procédé d'obtention de cellules de plantes transformées, caractérisé en ce qu'on insère dans le génome de la plante un ADNc codant pour la protéine selon les revendications 1 à 9.

18. Procédé selon la revendication 17, caractérisé en ce que l'ADNc est un ARN antisens de la protéine selon les revendications 1 à 9.

19. Cellules végétales obtenues par le procédé selon l'une des revendications 17 et 18

20. Plantes régénérées à partir de cellules selon la revendication 19.
